# EUROPÄISCHE PATENTSCHRIFT

(11) **EP 1 706 370 B1**
(45) Veröffentlichungstag und Bekanntmachung des Hinweises auf die Patenterteilung: **05.09.2007**
(21) Anmeldenummer: 05700737.9
(22) Anmeldetag: 07.01.2005
(51) Int. Cl.: C07C 209/84, C07C 211/50

(54) **VERFAHREN ZUR DESTILLATIVEN AUFBEREITUNG VON TOLUYLENDIAMIN**
METHOD FOR THE DISTILLATIVE RECOVERY OF TOLUYLENEDIAMINE
PROCEDE POUR LA PREPARATION PAR DISTILLATION DE TOLUYLENE DIAMINE

(30) Priorität: 08.01.2004 DE 102004001456
(43) Veröffentlichungstag der Anmeldung: 04.10.2006
(73) Patentinhaber: BASF Aktiengesellschaft, 67056 Ludwigshafen (DE)
(72) Erfinder: KNÖSCHE, Carsten, 67150 Niederkirchen (DE); SOHN, Martin, 68229 Mannheim (DE); PENZEL, Ulrich, 01945 Tettau (DE); PALLASCH, Hans-Jürgen, 67169 Kallstadt (DE); GEORGI, Gunter, 01979 Lauchhammer (DE); MACKENROTH, Wolfgang, 67089 Bad Dürkheim (DE); SCHWARZ, Hans Volkmar, B-3090 Overijse (BE); MAIXNER, Stefan, 68723 Schwetzingen (DE); MOLZ, Gerald, 67487 Maikammer (DE); STROEFER, Eckhard, 68163 Mannheim (DE)
(74) Vertreter: Isenbruck, Günter
(86) Internationale Anmeldenummer: PCT/EP2005/000081
(87) Internationale Veröffentlichungsnummer: WO 2005/066113

(56) Entgegenhaltungen:
- WO-A-94/06752
- DE-A1- 10 100 552
- GB-A- 1 303 562

## Beschreibung

Die Erfindung betrifft ein Verfahren zur destillativen Aufbereitung von Toluylendiamin aus einem Toluylendiamin enthaltenden Eduktstrom.

Toluylendiamin ist ein Zwischenprodukt bei der Herstellung von Toluylendiisocyanat. Dieses wird insbesondere als Monomer bei der Polyurethanherstellung verwendet. Weiterhin wird Toluylendiamin auch als Farbstoff zum Haare färben verwendet.

Bei den aus dem Stand der Technik bekannten Verfahren zur Reindestillation von Toluylendiamin (TDA) wird zunächst bei der Hydrierung von Dinitrotoluol anfallendes Roh-TDA einer Rektifikationskolonne zugeführt. In der Rektifikationskolonne werden die leicht siedenden Bestandteile über Kopf abgetrennt. Leicht siedende Bestandteile sind z.B. 3,4-TDA, ortho-Toluidin und Wasser. Dabei treten ortho-Toluidin und Wasser lediglich in Spuren auf. Das bei der Rektifikation anfallende Sumpfgemisch enthält insbesondere 2,4-TDA, 2,6-TDA und ein Oligomerengemisch, welches aus den im Sumpfgemisch enthaltenen TDA-Isomeren gebildet ist. Zur Abtrennung des Wertproduktes, enthaltend 2,4-TDA und 2,6-TDA, wird das Sumpfgemisch einem Dünnschichtverdampfer zugeführt. Im Dünnschichtverdampfer wird das Toluylen-Isomerengemisch abgetrennt. Ein solches Verfahren ist zum Beispiel in SRI-Report 1A, 1968, Seite 55 bis 65, beschrieben.

Eine weitere bekannte Möglichkeit, das TDA-Isomerengemisch aus dem Sumpfgemisch abzutrennen, ist der Einsatz einer zweiten Rektifikationskolonne. Hier wird das TDA-Isomerengemisch über Kopf abgezogen. Der verbleibende Sumpf, der vor allem das aus den TDA-Isomeren gebildete Oligomerengemisch und den Katalysator aus der Hydrierung enthält, wird einer geeigneten Entsorgung zugeführt.

WO 94/06752 beschreibt ein Verfahren zur Herstellung von rückstandsfreiem 2,4/2,6-Diaminotoluolgemisch.

Ein Nachteil der aus dem Stand der Technik bekannten Verfahren zur Reindestillation von TDA ist, dass zwei Apparate, zum Beispiel zwei Rektifikationskolonnen oder eine Rektifikationskolonne und ein Dünnschichtverdampfer, bereitgestellt werden müssen. Auch wird eine große Energiemenge zum Betrieb der Apparate benötigt.

Aufgabe der Erfindung war es, ein verbessertes, insbesondere wirtschaftlicheres Verfahren zur Reindestillation von TDA aus einem bei der Hydrierung von Dinitrotoluol anfallenden, TDA enthaltenden Eduktstromes bereitzustellen.

Die Lösung besteht in einem Verfahren zur Reindestillation von TDA aus einem TDA, Leichtsieder und Schwersieder enthaltenden Edukstrom in einer Trennwandkolonne, in welcher eine Trennwand in Kolonnenlängsrichtung unter Ausbildung eines oberen gemeinsamen Kolonnenbereiches, eines unteren gemeinsamen Kolonnenbereiches, eines Zulaufteils mit Verstärkungsteil und Abtriebsteil sowie eines Entnahmeteils mit Verstärkungsteil und Abtriebsteil angeordnet ist, welches folgende Schritte umfasst:
A) Zufuhr des Eduktstroms in den Zulaufteil der Trennwandkolonne,
B) Abziehen einer Leichtsiederfraktion über den Kolonnenkopf,
C) Abziehen von TDA über einen Seitenabzug im Entnahmeteil der Trennwandkolonne,
D) Abziehen einer Schwersiederfraktion über den Kolonnensumpf.

Der der Trennwandkolonne zugeführte Eduktstrom enthält vorzugsweise 80 bis 98 % TDA, 5 bis 15 % Leichtsieder und 0,5 bis 6 % Schwersieder, weiter bevorzugt 85 bis 96 % TDA, 5,5 bis 8,5 % Leichtsieder und 0,5 bis 3 % Schwersieder und besonders bevorzugt 90 bis 94 % TDA, 6 bis 6,5 % Leichtsieder und 1 bis 1,8 % Schwersieder.

Darin bedeutet TDA ein Isomerengemisch aus im wesentlichen 2,4-TDA und 2,6-TDA. Bevorzugt enthält das TDA-Isomerengemisch 70 bis 90 % 2,4-TDA und 10 bis 30 % 2,6-TDA, weiter bevorzugt 75 bis 85 % 2,4-TDA und 15 bis 25 % 2,6-TDA. Besonders bevorzugt enthält das TDA-Isomerengemisch 78 bis 82 % 2,4-TDA und 18 bis 22 % 2,6-TDA.

Der Leichtsieder setzt sich im Wesentlichen zusammen aus Vicinalen, Wasser und ortho-Toluidin. Dabei enthält der Leichtsieder bevorzugt 90 bis 100 % Vicinale, 0 bis 10 % Wasser und 0 bis 5 % ortho-Toluidin, weiter bevorzugt 92 bis 100 % Vicinale, 0 bis 7 % Wasser und 0 bis 2 % ortho-Toluidin und insbesondere 95 bis 100 % Vicinale, 0 bis 5 % Wasser und 0 bis 1 % ortho-Toluidin.

Darin bedeutet Vicinale ein Gemisch aus 2,3-TDA und 3,4-TDA. Bevorzugt ist eine Zusammensetzung aus 20 bis 50 % 2,3-TDA und 50 bis 80 % 3,4-TDA, weiter bevorzugt sind 30 bis 45 % 2,3-TDA und 55 bis 70 % 3,4-TDA und besonders bevorzugt sind 35 bis 40 % 2,3-TDA und 60 bis 65 % 3,4-TDA.

Der Schwersieder setzt sich im Wesentlichen aus Oligomeren und Polymeren zusammen, die durch Reaktion der TDA-Isomere miteinander entstehen. Die Oligomere und Polymere sind im Wesentlichen sekundäre bzw. tertiäre Amine. Die Oligomere und Polymere sind im wesentlichen Azo-, Azoxy- oder Hydrazin-Verbindungen.

Der Einsatz einer Trennwandkolonne ist zum Beispiel aus DE-A 101 00 552 für ein Verfahren zur destillativen Aufarbeitung von 1,6-Hexandiol, 1,5-Pentandiol und Caprolacton bekannt.
Eine Trennwandkolonne im Sinne der Erfindung ist eine Destillationskolonne mit mindestens einer senkrechten Trennwand, die in Teilbereichen eine Quervermischung von Flüssigkeits- und Brüdenströmen verhindert. Die mindestens eine Trennwand unterteilt die Kolonne in Längsrichtung in deren mittleren Bereich in einen Zulaufteil und ein Entnahmeteil.

In einer bevorzugten Ausführungsform ist die Trennwand als Blech aus einem metallischen Werkstoff gefertigt. Als metallische Werkstoffe eignen sich insbesondere eisenhaltige Stähle.

In einer weiteren Ausführungsform ist die Trennwand aus einem nicht-metallischen Werkstoff, zum Beispiel Keramik, gefertigt.

In einer weiteren bevorzugten Ausführungsform sind im gemeinsamen oberen Kolonnenbereich, im gemeinsamen unteren Kolonnenbereich, im Verstärkungsteil und Abtriebsteil des Zulaufsteils sowie im Verstärkungsteil und Abtriebsteil des Entnahmeteils Einbauten angeordnet. Als Einbauten eignen sich zum Beispiel Kolonnenböden, Füllkörperschüttungen oder strukturierte Packungen.

Bevorzugte Einbauten sind strukturierte Packungen oder Füllkörper. Davon sind besonders bevorzugte Blechpackungen oder Gewebepackungen.

Bei allen Einbauten ist darauf zu achten, dass diese druckverlustarm sind. Bevorzugt ist ein Druckverlust von weniger als 0,15 bar, mehr bevorzugt von weniger als 0,1 bar und besonders bevorzugt von weniger als 0,05 bar.

Die Füllkörperschüttungen und geordneten Packungen weisen vorzugsweise eine spezifische Oberfläche von 125 bis 500 m²/m³, besonders bevorzugt von 200 bis 300 m²/m³ auf.

In einer bevorzugten Ausführungsform weist die Trennwandkolonne 20 bis 50, besonders bevorzugt 25 bis 35 theoretische Trennstufen auf.

Die Aufteilung der Trennstufenzahl auf die einzelnen Teilbereiche der Trennwandkolonne erfolgt bevorzugt in der Weise, dass der gemeinsame obere Kolonnenbereich, das Verstärkungsteil und Abtriebsteil des Zulaufteils sowie der Verstärkungsteil und Abtriebsteil des Entnahmeteils jeweils 5 bis 50 %, bevorzugt 20 bis 40 % der Gesamtzahl der theoretischen Trennstufen der Trennwandkolonne aufweist. Der gemeinsame untere Kolonnenbereich weist bevorzugt 0 bis 30 % der Gesamtzahl der theoretischen Trennstufen der Trennwandkolonne auf, besonders bevorzugt ist der gemeinsame untere Kolonnenbereich der Kolonnensumpf.
In einer bevorzugten Ausführungsform erfolgt die Eduktzuführung über einen Seitenzulauf im Zulaufteil der Trennwandkolonne, welcher zwischen dem Zulaufteil und dem Verstärkungsteil angeordnet ist.

Zur Produktentnahme ist ein Seitenabzug im Entnahmeteil der Trennwandkolonne zwischen Abtriebsteil und Verstärkungsteil des Entnahmeteils angeordnet. Der Seitenabzug zur Produktentnahme ist dabei in einer Ausführungsform auf gleiche Höhe in der Trennwandklolonne angeordnet wie der Seitenzulauf zur Eduktzuführung.

In einer weiteren Ausführungsform ist der Seitenabzug zur Produktentnahme um 0 bis 20, bevorzugt um 5 bis 15 theoretische Trennstufen versetzt zum Seitenzulauf zur Eduktzuführung angeordnet.

In einer bevorzugten Verfahrensvariante ist die Flüssigkeitsverteilung in den einzelnen Teilbereichen der Trennwandkolonne jeweils getrennt einstellbar. Dadurch kann der gesamte Energiebedarf, der zur Auftrennung des Eduktstromes benötigt wird, minimiert werden.

Besonders vorteilhaft kann in den Teilbereichen des Zulaufteils der Trennwandkolonne die Flüssigkeit verstärkt im Wandbereich und in Teilbereichen der Trennwandkolonne reduziert im Wandbereich aufgegeben werden. Hierdurch werden unerwünschte Schleichströme vermieden und die erzielbaren Produkt-Endreinheiten gesteigert.

Die Trennwandkolonne kann in einen oder mehreren Teilbereichen mit geordneten Packungen oder Füllkörpern bestückt sein.

Es ist möglich, die Trennwand in Form von lose gesteckten Teilsegmenten auszuge-stalten. Dies führt zur weiteren Kostensenkung bei der Herstellung und Montage der Trennwandkolonne.

Besonders vorteilhaft kann die lose Trennwand interne Mannlöcher oder herausnehmbare Segmente aufweisen, die es erlauben, innerhalb der Trennwandkolonne von einer Seite der Trennwand auf die andere Seite zu gelangen.

Insbesondere für den Fall, dass Packungen als trennwirksame Einbauten eingesetzt werden, kann in einer weiteren Ausführungsform die Trennwand mit einer thermischen Isolierung ausgestattet sein. Besonders günstig ist eine doppelwandige Ausführung mit dazwischenliegendem engem Gasraum.

In einer bevorzugten Ausführungsform wird ein Teil der über den Kolonnensumpf abgezogenen Schwersiederfraktion über einen Seitenzulauf im unteren gemeinsamen Kolonnenbereich wieder der Trennwandkolonne zugeführt. Hierdurch wird gewährleistet, dass im Kolonnensumpf enthaltenes TDA erneut in die Trennwandkolonne gelangt und dort aus dem Kolonnensumpf verdampfen kann. Dies führt zu einer verbesserten Ausbeute an TDA, da weniger Wertprodukt über den Kolonnensumpf abgezogen wird. Der Teil der Schwersiederfraktion, der nicht der Trennwandkolonne erneut zugeführt wird, wird aus dem Destillationsverfahren abgezogen und bevorzugt einer Rezyklierung zugeführt.

In einer weiteren Verfahrensvariante wird ein Teil der über den Kolonnenkopf abgezogenen Leichtsiederfraktion über einen Seitenzulauf im oberen gemeinsamen Kolonnenbereich wieder der Trennwandkolonne zugeführt. Hierdurch wird eine weitere Abreicherung der Leichtsiederfraktion an TDA erreicht. Dies führt ebenso wie die Teilrückführung der über den Kolonnensumpf abgezogenen Schwersiederfraktion zu einer verbesserten Ausbeute am Wertprodukt TDA. Der nicht in die Trennwandkolonne zurückgeführte Teil der Leichtsiederfraktion wird aus dem Destillationsprozess abgezogen und vorzugsweise einer Rezyklierung zugeführt.

In einer bevorzugten Verfahrensvariante wird die Destillation des TDAs bei einem Druck im Kolonnensumpf, der unterhalb dem Umgebungsdruck, bevorzugt unterhalb 0,2 bar und insbesondere unterhalb 0,1 bar liegt, durchgeführt. Die Sumpftemperatur in der Trennwandkolonne liegt vorzugsweise unterhalb 250°C, mehr bevorzugt unterhalb 230°C und insbesondere unterhalb 220°C. Durch die Destillation bei einem Druck unterhalb des Umgebungsdruckes wird die Siedetemperatur des TDA gesenkt. Dies führt vorteilhafter Weise zu einer Einsparung an Heizenergie. Zudem wird hierdurch vermieden, dass das TDA zu Oligomeren oder Polymeren reagiert. Dies führt zu einer weiteren Verbesserung der Ausbeute an TDA bei der Reindestillation.

Ein weiterer Vorteil des Einsatzes der Trennwandkolonne zur Reindestillation von TDA im Vergleich zu den aus dem Stand der Technik bekannten Verfahren liegt darin, dass in der Trennwandkolonne bei gleicher Ausbeute ein geringerer hold-up realisiert werden kann. Dies führt dazu, dass weniger TDA oligomerisiert sowie dass bei der Destillation weniger TDA zu ortho-Toluidin reagiert. Hierdurch lässt sich mit der Trennwandkolonne eine größere Ausbeute an TDA erzielen als bei den aus dem Stand der Technik bekannten Verfahren.

Im Folgenden wird die Erfindung anhand einer Zeichnung näher beschrieben.

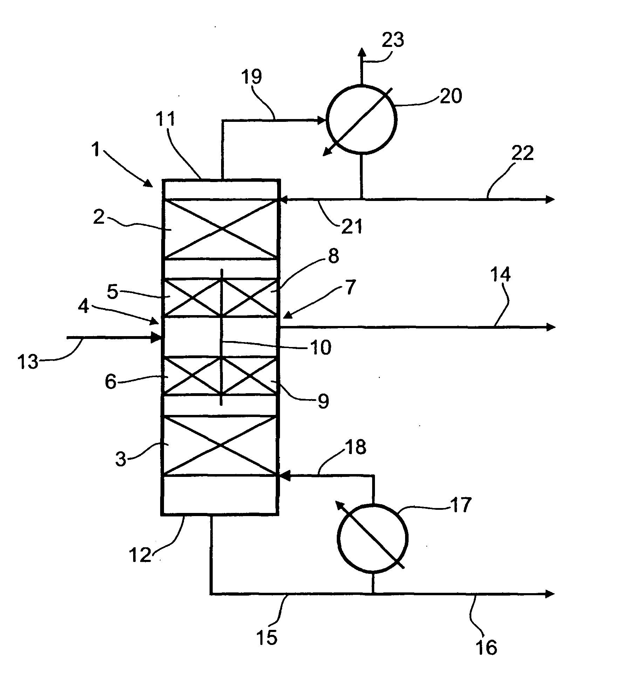

Darin zeigt die einzige Figur ein Verfahrensfließbild zur Reindestillation von TDA in einer Trennwandkolonne.

Eine Trennwandkolonne zur Durchführung des erfindungsgemäßen Verfahrens zur Reindestillation von TDA umfasst einen oberen gemeinsamen Kolonnenbereich 2, einen unteren gemeinsamen Kolonnenbereich 3, einen Zulaufteil 4 und einen Entnahmeteil 7. Bei der in der Figur dargestellten Ausführungsform ist der Zulaufteil 4 in einen Verstärkungsteil 5 und einen Abtriebsteil 6 aufgeteilt, der Entnahmeteil 7 in einen Abtriebsteil 8 und einen Verstärkungsteil 9. Innerhalb der Trennwandkolonne 1 sind der Zulaufteil 4 und der Entnahmeteil 7 durch eine Trennwand 10 getrennt.

Die Trennwand 10 ist vorzugsweise aus einem metallischen Werkstoff, insbesondere aus Edelstahl gefertigt. Neben metallischen Werkstoffen eignet sich jedoch auch Keramik zur Fertigung der Trennwand 10.

Zum Betrieb der Trennwandkolonne 1 wird im Zulaufteil 4 ein Eduktstrom 13 zugeführt. Der Zulauf des Eduktstromes 13 erfolgt vorzugsweise zwischen dem Verstärkungsteil 5 und dem Abtriebsteil 6 des Zulaufteils 4.

Der Eduktstrom 13 fällt bei der Hydrierung von Dinitrotoluol an und enthält ein Isomerengemisch aus im Wesentlichen 2,4-TDA und 2,6-TDA, Vicinale, ein aus TDA gebildetes Oligomerengemisch sowie ortho-Toluidin und Wasser.

Zur verbesserten destillativen Auftrennung des Eduktstromes 13 sind im oberen gemeinsamen Kolonnenbereich 2, im unteren gemeinsamen Kolonnenbereich 3, im Verstärkungsteil 5 und Abtriebsteil 6 des Zulaufteils 4 sowie im Verstärkungsteil 9 und Abtriebsteil 8 des Entnahmeteils 7 Einbauten vorgesehen. Als Einbauten eignen sich zum Beispiel Böden, strukturierte Packungen oder Füllkörper.

Am Entnahmeteil 7 ist vorzugsweise zwischen dem Abtriebsteil 8 und dem Verstärkungsteil 9 ein Seitenabzug angeordnet, über welchen ein Produktstrom 14 abgezogen wird. Der Produktstrom 14 enthält ein Isomerengemisch aus im Wesentlichen 2,4-TDA und 2,6-TDA.

Der Seitenabzug für den Produktstrom 14 ist auf gleicher Höhe wie der Seitenzulauf für den Eduktstrom 13 oder vorzugsweise versetzt zur Höhe des Seitenzulaufs für den Eduktstrom 13 angeordnet.

Während der Destillation reichert sich im Sumpf 12 der Trennwandkolonne 1 die im Eduktstrom 13 enthaltenen Hochsieder an. Die im Eduktstrom 13 enthaltenen Schwersieder sind z.B. das aus TDA gebildete Oligomerengemisch.

Der Schwersieder wird als Sumpfstrom 15 aus dem Kolonnensumpf 12 abgezogen. Über eine erste Pumpe 17 wird ein Teilstrom 18 des Schwersieders vorzugsweise über einen Seitenzulauf dem unteren gemeinsamen Kolonnenbereich 3 erneut zugeführt. Der nicht erneut der Trennwandkolonne 1 zugeführte Anteil wird über einen Schwersiederabzug 16 aus dem Destillationsprozess entfernt.

Über den Kolonnenkopf 11 der Trennwandkolonne 1 wird ein Leichtsieder enthaltender Kopfstrom 19 abgezogen. Als Leichtsieder sind im Kopfstrom 19 insbesondere Vecinale, ortho-Toluidin und Wasser enthalten. Der Kopfstrom 19 wird einer Pumpe 20 zugeführt. Bei der in der Figur dargestellten Ausführungsform wird an der zweiten Pumpe 20 ein Gasstrom 23 aus dem Kopfstrom 19 abgezogen. Der restliche Kopfstrom 19 wird hinter der zweiten Pumpe 20 aufgeteilt. Ein Teilstrom 21 wird vorzugsweise über einen Seitenzulauf dem oberen gemeinsamen Kolonnenbereich 2 und damit erneut der Destillation zugeführt. Der restliche Leichtsieder wird über einen Leichtsiederabzug 22 aus dem Destillationsprozess abgezogen.

### Beispiel

Einer Trennwandkolonne wird über einen Seitenzulauf ein bei der Hydrierung von Dinitroluol anfallender Massenstrom von 5.000 kg/h zugeführt. Der Eduktstrom enthält 92,37 Gew.-% TDA, 6,02 Gew.-% 3,4-TDA, 0,05 Gew.-% o-Toluidin, 0,13 Gew.-% Wasser und 1,43 Gew.-% Schwersieder. Die Destillation wird bei einem Druck im Kolonnensumpf von 0,07 bar und einer Sumpftemperatur von 215°C durchgeführt. Im Kolonnenkopf stellt sich ein Druck von 0,07 bar bei einer Temperatur von 140°C ein. Das 3,4-TDA, das o-Toluidin und das Wasser werden über den Kolonnenkopf abgezogen. Über den Seitenabzug im Entnahmeteil werden 4.602 kg/h Produktstrom abgezogen. Der Produktstrom setzt sich zusammen aus 99,95 Gew.-% TDA-Isomerengemisch und 0,05 Gew.-% Leichtsieder. Das TDA-Isomerengemisch enthält 80 Gew.-% 2,4-TDA und 20 Gew.-% 2,6-TDA.

### Bezugszeichenliste

- 1: Trennwandkolonne
- 2: obere gemeinsamer Kolonnenbereich
- 3: unterer gemeinsamer Kolonnenbereich
- 4: Zulaufteil
- 5: Verstärkungsteil
- 6: Abtriebsteil
- 7: Entnahmeteil
- 8: Abtriebsteil
- 9: Verstärkungsteil
- 10: Trennwand
- 11: Kolonnenkopf
- 12: Kolonnensumpf
- 13: Eduktstrom
- 14: Produktstrom
- 15: Sumpfstrom
- 16: Schwersiederabzug
- 17: erste Pumpe
- 18: Schwersiederteilstrom
- 19: Kopfstrom
- 20: zweite Pumpe
- 21: Leichtsiederteilstrom
- 22: Leichtsiederabzug
- 23: Gasstrom

## Patentansprüche

1. Verfahren zur destillativen Aufbereitung von TDA aus einem TDA, Schwersieder und Leichtsieder enthaltenden Eduktstrom in einer Trennwandkolonne, in welcher eine Trennwand in Kolonnenlängsrichtung unter Ausbildung eines oberen gemeinsamen Kolonnenbereiches (2), eines unteren gemeinsamen Kolonnenbereiches (3), eines Zulaufteils (4) mit Verstärkungsteil (5) und Abtriebsteil (6) sowie eines Entnahmeteils (7) mit Verstärkungsteil (9) und Abtriebsteil (8) angeordnet ist, welches folgende Schritte umfasst:
A) Zufuhr des Eduktstroms (13) in den Zulaufteil (4) der Trennwandkolonne (1);
B) Abziehen einer Leichtsiederfraktion über den Kolonnenkopf (11);
C) Abziehen von TDA über einen Seitenabzug (14) im Entnahmeteil (7) der Trennwandkolonne (1);
D) Abziehen einer Schwersiederfraktion über den Kolonnensumpf (12).

2. Verfahren nach Anspruch 1, **dadurch gekennzeichnet, dass** ein Teil der über den Kolonnensumpf (12) abgezogenen Schwersiederfraktion über einen Seitenzulauf im unteren gemeinsamen Kolonnenbereich (3) wieder der Trennwandkolonne (1) zugeführt wird.

3. Verfahren nach Anspruch 1 oder 2, **dadurch gekennzeichnet, dass** ein Teil der über den Kolonnenkopf (11) abgezogenen Leichtsiederfraktion über einen Seitenzulauf im oberen gemeinsamen Kolonnenbereich (2) wieder der Trennwandkolonne (1) zugeführt wird.

4. Verfahren nach einem der Ansprüche 1 bis 3, **dadurch gekennzeichnet, dass** die Eduktzuführung und der Seitenabzug zur Produktentnahme auf gleicher Höhe in der Trennwandkolonne (1) angeordnet sind.

5. Verfahren nach einem der Ansprüche 1 bis 3, **dadurch gekennzeichnet, dass** die Eduktzuführung und der Seitenabzug zur Produktentnahme in unterschiedlicher Höhe in der Trennwandkolonne (1) angeordnet sind.

6. Verfahren nach Anspruch 5, **dadurch gekennzeichnet, dass** der Seitenabzug zur Produktentnahme um 5 bis 15 theoretische Trennstufen versetzt zur Eduktführung angeordnet ist.

7. Verfahren nach einem der Ansprüche 1 bis 6, **dadurch gekennzeichnet, dass** die Destillation bei einem Druck im Kolonnensumpf von ≤ 1 bar durchgeführt wird.

8. Verfahren nach einem der Ansprüche 1 bis 7, **dadurch gekennzeichnet, dass** die Destillation bei einem Druck im Kolonnensumpf von ≤ 0,2 bar durchgeführt wird.

9. Verfahren nach einem der Ansprüche 1 bis 8, **dadurch gekennzeichnet, dass** die Destillation bei einem Druck im Kolonnensumpf von ≤ 0,1 bar durchgeführt wird.

10. Verfahren nach einem der Ansprüche 1 bis 9, **dadurch gekennzeichnet, dass** die Sumpftemperatur unterhalb von 250°C liegt.

11. Verfahren nach einem der Ansprüche 1 bis 10, **dadurch gekennzeichnet, dass** die Sumpftemperatur unterhalb 230°C liegt.

12. Verfahren nach einem der Ansprüche 1 bis 11, **dadurch gekennzeichnet, dass** die Sumpftemperatur unterhalb 220°C liegt.

## Claims

1. A process for distillatively preparing TDA from a reactant stream comprising TDA, high boilers and low boilers in a dividing wall column in which a dividing wall is disposed in the longitudinal direction of the column to form an upper combined column region (2), a lower combined column region (3), a feed section (4) having a rectifying section (5) and stripping section (6), and also a withdrawal section (7) having a rectifying section (9) and stripping section (8), which comprises the following steps:
A) feeding the reactant stream (13) into the feed section (4) of the dividing wall column (1);
B) drawing off a low boiler fraction via the top of the column (11);
C) drawing off TDA via a side draw (14) in the withdrawal section (7) of the dividing wall column (1);
D) drawing off a low boiler fraction via the bottom of the column (12).

2. The process according to claim 1, wherein a portion of the high boiler fraction drawn off via the bottom of the column (12) is fed back to the dividing wall column (1) via a side feed in the lower combined column region (3).

3. The process according to claim 1 or 2, wherein a portion of the low boiler fraction drawn off via the top of the column (11) is fed back to the dividing wall column (1) via a side feed in the upper combined column region (2).

4. The process according to any of claims 1 to 3, wherein the reactant feed and the side draw for product withdrawal are disposed at the same height in the dividing wall column (1).

5. The process according to any of claims 1 to 3, wherein the reactant feed and the side draw for product withdrawal are disposed at different height in the dividing wall column (1).

6. The process according to claim 5, wherein the side draw for product withdrawal is offset by from 5 to 15 theoretical plates from the reactant feed.

7. The process according to any of claims 1 to 6, wherein the distillation is carried out at a pressure in the column bottom of ≤ 1 bar.

8. The process according to any of claims 1 to 7, wherein the distillation is carried out at a pressure in the column bottom of ≤ 0.2 bar.

9. The process according to any of claims 1 to 8, wherein the distillation is carried out at a pressure in the column bottom of ≤ 0.1 bar.

10. The process according to any of claims 1 to 9, wherein the bottom temperature is below 250°C.

11. The process according to any of claims 1 to 10, wherein the bottom temperature is below 230°C.

12. The process according to any of claims 1 to 11, wherein the bottom temperature is below 220°C.

## Revendications

1. Procédé pour la préparation par distillation de TDA à partir d'un courant d'éduit contenant du TDA, une fraction à haut point d'ébullition et une fraction à bas point d'ébullition dans une colonne à paroi de séparation, dans laquelle une paroi de séparation est disposée dans le sens de la longueur de la colonne avec formation d'une section de colonne supérieure commune (2), d'une section de colonne inférieure commune (3), d'une partie d'admission (4) avec une partie de renforcement (5) et une partie de fractionnement (6), de même que d'une partie de prélèvement (7) avec une partie de renforcement (9) et une partie de fractionnement (8), qui comprend les étapes suivantes :
A) Amenée du courant d'éduit (13) dans la partie d'admission (4) de la colonne à paroi de séparation (1) ;
B) Extraction d'une fraction à bas point d'ébullition via la tête de la colonne (11) ;
C) Extraction de TDA via une extraction latérale (14) dans la partie de prélèvement (7) de la colonne à paroi de séparation (1) ;
D) Extraction d'une fraction à haut point d'ébullition via le fond de la colonne (12).

2. Procédé selon la revendication 1, **caractérisé en ce qu'**une partie de la fraction à haut point d'ébullition extraite via le fond de la colonne (12) est ramenée via une admission latérale dans la section de colonne inférieure commune (3) à la colonne à paroi de séparation (1).

3. Procédé selon la revendication 1 ou 2, **caractérisé en ce qu'**une partie de la fraction à bas point d'ébullition extraite via la tête de la colonne (11) est ramenée via une admission latérale dans la section de colonne supérieure commune (2) à la colonne à paroi de séparation (1).

4. Procédé selon une des revendications 1 à 3, **caractérisé en ce que** l'amenée d'éduit et l'extraction latérale pour le prélèvement de produit sont disposées à la même hauteur dans la colonne à paroi de séparation (1).

5. Procédé selon une des revendications 1 à 3, **caractérisé en ce que** l'amenée d'éduit et l'extraction latérale pour le prélèvement de produit sont disposées à des hauteurs différentes dans la colonne à paroi de séparation (1).

6. Procédé selon la revendication 5, **caractérisé en ce que** l'extraction latérale pour le prélèvement de produit est décalée de 5 à 15 étapes de séparation théoriques par rapport à l'amenée d'éduit.

7. Procédé selon une des revendications 1 à 6, **caractérisé en ce que** la distillation est exécutée sous une pression ≤ 1 bar dans le fond de la colonne.

8. Procédé selon une des revendications 1 à 7, **caractérisé en ce que** la distillation est exécutée sous une pression ≤ 0,2 bar dans le fond de la colonne.

9. Procédé selon une des revendications 1 à 8, **caractérisé en ce que** la distillation est exécutée sous une pression ≤ 0,1 bar dans le fond de la colonne.

10. Procédé selon une des revendications 1 à 9, **caractérisé en ce que** la température du fond est inférieure à 250°C.

11. Procédé selon une des revendications 1 à 10, **caractérisé en ce que** la température du fond est inférieure à 230°C.

12. Procédé selon une des revendications 1 à 11, **caractérisé en ce que** la température du fond est inférieure à 220°C.
